# EUROPEAN PATENT APPLICATION

(11) **EP 4 729 050 A1**
(43) Date of publication of application: **22.04.2026**
(21) Application number: 25209130.1
(22) Date of filing: 16.10.2025
(51) Int. Cl.: A61K 9/16, A61K 9/48, A61K 9/50, A61K 31/616, A61P 9/10

(54) **PHARMACEUTICAL COMPOSITION COMPRISING TICAGRELOR AND ACETYLSALICYLIC ACID, AND PROCESS FOR PREPARING THE SAME**

(30) Priority: 17.10.2024 MX 2024012886
(71) Applicant: Laboratorios Silanes, S.A. de C.V., Miguel Hidalgo, Ciudad de México 11000 (MX)
(72) Inventor: Ollervides Rubio, Paola Yazmín, 11000 México (MX); Cuahutencos Escobar, Ernesto, 11000 México (MX); González Canudas, Jorge Alejandro, 11000 México (MX); Rios Brito, Kevin Francisco, 11000 México (MX)
(74) Representative: Hoffmann Eitle

(57) **Abstract**

The present invention provides a pharmaceutical composition comprising ticagrelor at a dose of 90 mg, acetylsalicylic acid at a dose of from 75 to 100 mg, at least one of a diluent, a binder, a wetting agent, a glidant, and a lubricant. The pharmaceutical composition of the present invention solves a set of significant technological challenges due to the physicochemical properties and difference in dosage of each drug. Said pharmaceutical composition is stable, such that the composition is safe and effective in the treatment and prevention of cardiovascular diseases. The invention is further directed to a process for manufacturing a pharmaceutical composition.

## Description

### TECHNICAL FIELD

The present invention relates to the field of the pharmaceutical industry and more particularly to a pharmaceutical composition comprising ticagrelor and acetylsalicylic acid, a process for preparing the same, and uses thereof.

### BACKGROUND

Cardiovascular diseases (CVD) are one of the leading causes of mortality and morbidity worldwide. This category includes various pathologies that affect the heart and blood vessels such as coronary heart disease, heart failure, hypertensive heart disease, among others. CVD has a multifactorial etiology, involving both genetic and environmental factors.

Major modifiable risk factors include smoking, high blood pressure, hypercholesterolemia, diabetes mellitus, obesity, physical inactivity, and excessive alcohol consumption. Added to these are non-modifiable factors such as age, gender, and family history of cardiovascular disease.

Atherosclerosis, characterized by the accumulation of lipids in the artery walls, is one of the main underlying causes of many CVDs. This chronic inflammatory process can begin in youth and progress throughout life, being exacerbated by the aforementioned risk factors.

The treatment of CVDs varies depending on the type and severity of the disease and includes both pharmacological and non-pharmacological interventions. Among the most common therapeutic options are:
Medicaments: Antihypertensives, antiplatelet agents, statins, anticoagulants, antiarrhythmics, and other specific drugs depending on the pathology.
Interventional procedures: Angioplasty, stent graft placement or stenting, and coronary artery bypass surgery are options for treating significant blockages in the arteries.
Cardiac rehabilitation: Planned programs that include supervised exercise, disease management education, and psychological support.
Implants and devices: In cases of severe arrhythmias, pacemakers and implantable defibrillators may be used.

Acute arterial thrombo-occlusive events often occur in areas affected by atherosclerotic disease following spontaneous plaque rupture; in said areas, platelet activation leads to the formation of white thrombi that can occlude vascular blood flow or detach, causing embolisms that occlude blood flow in a more distal region.

Arterial occlusion by a thrombus is the usual cause of myocardial infarctions, strokes, and acute peripheral ischemic infarctions. In addition, platelet-dependent thrombo-occlusive events complicate the interventional techniques used in the treatment of patients with symptomatic atherosclerotic disease, such as thrombolytic reperfusion in acute myocardial infarction, endarterectomy, and small-caliber vascular graft implantation.

Consequently, antiplatelet agents are crucial for preventing and treating cardiovascular diseases, including ischemic heart disease, ischemic cerebrovascular disease, and peripheral artery disease.

An exemplary antiplatelet agent is acetylsalicylic acid (ASA) or aspirin, which is considered the "gold standard" and acts by irreversibly inactivating platelet cyclooxygenase, thereby inhibiting platelet synthesis of thromboxane A2 (TXA2) and thus partially blocking the final stage of aggregation. Exposure of platelets to a single dose of ASA impairs their function throughout platelet lifetime (seven to ten days).

Additionally, ticagrelor and Prasugrel are considered to be the most potent antiplatelet agents and are therefore used as first-line treatment, relegating clopidogrel to events where there is a higher risk of bleeding or intolerance, cases where they cannot be used, or where there is a lack of current evidence (such as concomitant use with anticoagulants).

To improve and optimize the treatment of CVDs, therapies involving two antiplatelet agents have been suggested, such as dual antiplatelet therapy with ASA and clopidogrel.

Clopidogrel is a prodrug that needs to be metabolized in the liver so that the active metabolite can bind to ADP receptors (P2Y12), leading to an irreversible modification of the receptor throughout the platelet lifetime. However, variability in the response to clopidogrel has been detected due to polymorphisms in the enzymes involved in its hepatic metabolism.

In order to overcome the limitation represented by "resistance" to clopidogrel, combination therapies of ASA with ticagrelor are recommended. Ticagrelor does not require hepatic metabolism and inhibits platelet activation and aggregation by reversibly interacting with the platelet P2Y12 receptor for adenosine diphosphate (ADP) to prevent signal transduction.

To improve patient adherence to treatment, simplify medication regimen, reduce dosing errors, and even decrease the side effects of medicaments, it is preferable to have single formulations that comprise the active ingredients involved in a treatment, in this particular case, two antiplatelet agents.

However, combining two antiplatelet agents, particularly acetylsalicylic acid and ticagrelor, poses a technological challenge due to the difference in physicochemical properties of the active ingredients. On the one hand, acetylsalicylic acid is a highly unstable drug in the presence of water in the environment, while ticagrelor is a drug with very low solubility and high adhesiveness. To solve this problem, technologies have been developed to reduce the particle size of ticagrelor or provide products comprising several layers with the drugs.

In this regard, document CN114533744 discloses a ticagrelor-aspirin compound granule preparation comprising enteric-coated aspirin granules, fast-release ticagrelor granules, and sustained-release ticagrelor granules. Similarly, document CN114533694 discloses a composition comprising ticagrelor and aspirin, wherein ticagrelor is contained in a sustained-release tablet while aspirin is contained in an enteric-coated tablet.

International Publication No. WO2014191321 discloses a solid pharmaceutical dosage form comprising different first and second compartments, wherein said first compartment contains the pharmaceutically active agent ticagrelor or a pharmaceutically acceptable salt, solvate or ester thereof, and said second compartment contains the pharmaceutically active agent acetylsalicylic acid or a pharmaceutically acceptable salt, solvate or ester thereof.

Likewise, document CN104971070 discloses an oral nanocomposition of ticagrelor comprising ticagrelor, aspirin, an emulsifier, a co-emulsifier, and an oil phase.

Documents CN104434931 and CN107260700 suggest that, to avoid stability problems, the active ingredients should not come into direct contact with each other and, in particular, disclose a solid pharmaceutical composition wherein acetylsalicylic acid has an enteric coating.

Document CN111939136 discloses a composition comprising two layers. The first one is a fast-release layer and comprises an enteric-coated acetylsalicylic acid tablet with a core comprising a solid fast-release ticagrelor composition. The second one is a sustained-release layer and corresponds to a solid ticagrelor composition.

Document IN202011032760 discloses a composition comprising ticagrelor and aspirin, wherein ticagrelor is formulated as sustained-release coated granules, and aspirin is formulated as enteric-coated granules.

None of the documents described above propose a pharmaceutical composition wherein ticagrelor and acetylsalicylic acid interact in the form of granules in the same compartment without the addition of enteric coatings, while providing immediate release of the drugs as claimed in the present invention.

As a result of the above, efforts have been made to eliminate the drawbacks related to stability of pharmaceutical compositions comprising ticagrelor and acetylsalicylic acid as active ingredients by developing a pharmaceutical composition comprising ticagrelor and acetylsalicylic acid, a process for preparing the same, and uses thereof, wherein a single, solid, stable, immediate-release pharmaceutical form is provided. The proposed composition and manufacturing process thereof overcome the major drawbacks associated with the combination of active ingredients through its novel manufacturing process.

Thus, the present invention proposes a combination of the active ingredients ticagrelor and acetylsalicylic acid into a single dosage unit, thereby solving a set of significant technological challenges due to the physicochemical properties and difference in dosage to ensure obtaining a stable product for treating and preventing cardiovascular diseases.

### OBJECTS OF THE INVENTION

Considering the shortcomings of the prior art, it is an object of the present invention to provide a solid pharmaceutical composition comprising ticagrelor and acetylsalicylic acid, wherein said pharmaceutical composition is stable, immediate release, and easy to administer.

A further object of the invention is to provide a process for manufacturing a pharmaceutical composition comprising ticagrelor and acetylsalicylic acid, improving the stability of the active ingredients.

These and other objects are achieved by a pharmaceutical composition comprising ticagrelor and acetylsalicylic acid, a process for preparing the same, and uses thereof according to the present invention.

### SUMMARY OF THE INVENTION

To this end, a first aspect of the present invention relates to a pharmaceutical composition comprising:
- ticagrelor at a dose of 90 mg;
- acetylsalicylic acid at a dose of from 75 to 100 mg;
- at least one diluent in an amount of from 5 to 30% by weight of the composition;
- at least one binder in an amount of from 1 to 8% by weight of the composition;
- a wetting agent in an amount of from 1 to 2.5% by weight of the composition;
- a glidant in an amount of from 0.5% to 2% by weight of the composition; and
- a lubricant in an amount of from 0.5% to 1.5% by weight of the composition.

In a second aspect of the invention, a process for manufacturing the pharmaceutical composition described above is provided, wherein the process comprises the following stages:
a) providing a first granulate comprising ticagrelor and pharmaceutically acceptable excipients;
b) providing a second granulate comprising acetylsalicylic acid and pharmaceutically acceptable excipients;
c) mixing the first granulate comprising ticagrelor and the second granulate comprising acetylsalicylic acid, thereby obtaining a third granulate comprising ticagrelor, acetylsalicylic acid, and pharmaceutically acceptable excipients; and
d) lubricating the third granulate.

A third aspect of the invention is directed to the use of the pharmaceutical composition of the present invention in the treatment and prevention of cardiovascular diseases.

### BRIEF DESCRIPTION OF THE DRAWINGS

The novel aspects considered as characteristic of the present invention will be set forth particularly in the appended claims. However, some embodiments, features, and some objects and advantages thereof will be better understood from the detailed description when read in conjunction with the accompanying drawings, in which:
Figure 1 depicts the dissolution profile of acetylsalicylic acid.
Figure 2 shows the dissolution profile of ticagrelor.

### DETAILED DESCRIPTION

It should be understood that the present invention is not limited to the methodologies, protocols, and reagents provided by the detailed description, as these may vary. It should also be understood that the terminology used herein is merely for describing particular embodiments and is not intended to limit the scope of the present disclosure, which will only be limited by the accompanying claims. Unless otherwise defined, all technical and scientific terms used herein have the same meaning as those commonly understood by a person skilled in the art.

As used in the present invention, the term "approximately" refers to a measurable value that, due to the nature of the techniques used to measure it, may vary. In the case of an amount of weight, time, dose, etc., it is understood to encompass, for example, deviations of ± 20% or ± 10%, as another example ± 5%, as another example ± 1%, and as another example ± 0.1% from a specified amount, as such deviations are appropriate for carrying out the disclosed process.

The term "pharmaceutically acceptable salt" of a given compound refers to salts that retain the biological efficacy and properties of the given compound and are not biologically or otherwise undesirable. Pharmaceutically acceptable base addition salts can be prepared from inorganic or organic bases. Salts derived from inorganic bases include, by way of example only, sodium, potassium, lithium, ammonium, calcium, and magnesium salts.

As used herein, the term "stability" is defined as the ability of a pharmaceutical product to retain its chemical, physical, microbiological, and biopharmaceutical properties within specified limits throughout its shelf-life.

The present invention relates to solid, stable, immediate-release pharmaceutical compositions that maintain the dissolution and bioavailability of two antiplatelet agents. Specifically, in the compositions of the present invention, the two antiplatelet agents correspond to ticagrelor and acetylsalicylic acid. In this regard, it is known that the combination of ticagrelor and acetylsalicylic acid presents a set of significant technological challenges because the drugs have different physicochemical properties. Specifically, ticagrelor is a drug with very low solubility and high adhesiveness, while acetylsalicylic acid is a drug that is highly unstable in the presence of water in the environment.

The combination of ticagrelor and acetylsalicylic acid requires special care to ensure that there is no interaction between the drugs because ticagrelor has low solubility and could interfere with the absorption process of acetylsalicylic acid. Thus, the aim is to keep side effects to a minimum while achieving the maximum benefit from both drugs.

In order to solve the technical problem described above, the present invention provides a pharmaceutical composition comprising:
- ticagrelor at a dose of 90 mg;
- acetylsalicylic acid at a dose of from 75 to 100 mg;
- at least one diluent in an amount of from 5 to 30% by weight of the composition;
- at least one binder in an amount of from 1 to 8% by weight of the composition;
- a wetting agent in an amount of from 1 to 2.5% by weight of the composition;
- a glidant in an amount of from 0.5% to 2% by weight of the composition; and
- a lubricant in an amount of from 0.5% to 1.5% by weight of the composition.

In one embodiment, the at least one diluent is selected from, but not limited to, the group consisting of starch derivatives such as pregelatinized starch and corn starch; cellulose derivatives such as microcrystalline cellulose; mannitol; maltitol; dextrose; dibasic calcium phosphate; and combinations thereof.

In another embodiment, the at least one binder is selected from, but not limited to, the group consisting of cellulose derivatives such as hydroxypropyl cellulose and carboxymethyl cellulose; starch derivatives such as pregelatinized starch and corn starch; polyvinylpyrrolidone; glyceryl dibehenate; povidones; polyethylene glycols; poloxamers; and combinations thereof.

In another embodiment, the wetting agent is selected from, but not limited to, the group consisting of poloxamers; sodium lauryl sulfate; castor oil derivatives; benzalkonium chloride; benzetonium chloride; polyoxyethylene alkyl esters; polyoxyethylene sorbitan fatty acid esters; and combinations thereof.

In another embodiment, the glidant is selected from, but not limited to, the group consisting of cellulose derivatives such as carboxymethyl cellulose and microcrystalline cellulose; starch derivatives such as pregelatinized starch and corn starch; silicate derivatives such as magnesium silicate and magnesium trisilicate; talc; colloidal silicon dioxide; and combinations thereof.

In a further embodiment, the lubricant is selected from, but not limited to, the group consisting of stearate derivatives such as magnesium stearate, zinc stearate, calcium stearate, stearic acid, monostearates, and stearyl fumarate; sulfated derivatives such as magnesium lauryl sulfate and sodium lauryl sulfate; talc; and combinations thereof.

In a preferred embodiment, the pharmaceutical composition comprises two diluents.

In another preferred embodiment, the pharmaceutical composition comprises two binders.

In a preferred embodiment, the pharmaceutical composition comprises:
- ticagrelor at a dose of 90 mg;
- acetylsalicylic acid at a dose of from 75 to 100 mg;
- pregelatinized starch as a first diluent in an amount of from 20% to 30% by weight of the composition;
- hydroxypropyl cellulose as a first binder in an amount of from 2% to 3% by weight of the composition;
- glyceryl dibehenate as a second binder in an amount of from 1% to 2% by weight of the composition;
- sodium lauryl sulfate as a wetting agent in an amount of from 1.5% to 2.5% by weight of the composition;
- colloidal silicon dioxide as a glidant in an amount of from 0.5% to 2% by weight of the composition;
- microcrystalline cellulose as a second diluent in an amount of from 10% to 15% by weight of the composition; and
- zinc stearate as a lubricant in an amount of from 0.5% to 1% by weight of the composition.

In a more preferred embodiment, the pharmaceutical composition as described above is in the pharmaceutical form of capsule, which comprises a pharmaceutically acceptable vehicle. The pharmaceutically acceptable vehicle is a capsule, preferably a size #0 capsule with opaque orange body and opaque orange cap.

In an even more preferred embodiment, the pharmaceutical composition has a qualitative and quantitative formula as described in Table 1.

**Table 1. Qualitative and quantitative formula of the pharmaceutical composition**

| **Components** | **Function in the formulation** | **Concentration in the formulation (%)** |
|---|---|---|
| Ticagrelor⁽¹⁾ | Drug | 16.36 |
| Acetylsalicylic acid⁽¹⁾ | Drug | 18.18 |
| Pregelatinized starch⁽²⁾ | Diluent | 26.53 |
| Hydroxypropyl cellulose EF | Binder | 2.73 |
| Glyceryl dibehenate | Binder | 1.82 |
| Sodium lauryl sulfate | Wetting agent | 2.00 |
| Colloidal silicon dioxide | Glidant | 0.85 |
| Microcrystalline cellulose PH112 | Diluent | 13.49 |
| Zinc stearate | Lubricant | 0.55 |
| Purified water ⁽³⁾ | Solvent | q.s. |
| #0 Capsule with opaque orange body and opaque orange cap | Vehicle | 17.45 |

| | | |
|---|---|---|
| ⁽¹⁾ Adjustment is made for purity ⁽²⁾ It is adjusted to total solids ⁽³⁾ It evaporates during the process | | |

The pharmaceutical composition as described above is notable for its physical, chemical, and biological stability, as well as for its controlled drug release and low cost.

Surprisingly, the pharmaceutical composition of the present application was found to be highly stable, since, in a 6-month accelerated stability study, the pharmaceutical composition of the present invention was found to retain its chemical and physical characteristics within the established specifications, maintaining its quality, efficacy, and safety.

Additionally, the dissolution of the active ingredients ticagrelor and acetylsalicylic acid contained in the pharmaceutical composition of the present invention was found to be comparable to the dissolution profiles of the reference drugs, and even the dissolution of the drugs in the pharmaceutical composition of the present invention is better compared to the reference drugs. This has an impact on the medicament efficacy, as it would positively affect the drug bioavailability.

The pharmaceutical composition of the present invention is useful in the prevention and treatment of cardiovascular diseases. In this regard, it has been described that arterial occlusion by a thrombus is the usual cause of myocardial infarctions, strokes, and acute peripheral ischemic infarctions. In addition, platelet-dependent thrombo-occlusive events complicate the interventional techniques used in the treatment of patients with symptomatic atherosclerotic disease, such as thrombolytic reperfusion in acute myocardial infarction, endarterectomy, and small-caliber vascular graft implantation.

Consequently, the administration of antiplatelet agents is useful in the prevention and treatment of cardiovascular diseases, including ischemic heart disease, ischemic cerebrovascular disease, and peripheral artery disease. Furthermore, the combination of ticagrelor and acetylsalicylic acid provides a greater effect in the prevention and treatment of atherothrombotic events in patients with acute coronary syndromes (ACS) or a history of myocardial infarction (MI) and a high risk of developing an atherothrombotic event, compared to the administration of a single antiplatelet agent.

Accordingly, the present invention also provides a method for preventing and treating cardiovascular diseases, wherein the cardiovascular disease is selected from the group consisting of coronary heart disease, heart failure, arrhythmia, cerebrovascular disease, peripheral artery disease, congenital heart disease, cardiomyopathy, valvular heart disease, and for angioplasty procedures.

In another aspect of the invention, a process for manufacturing the pharmaceutical composition described above is provided. The manufacturing process is advantageous over others described in the prior art, as it involves simple and efficient unit operations that ensure obtaining a reproducible and stable product from batch to batch.

The process for manufacturing the pharmaceutical composition of the present invention comprises the stages of:
a) providing a first granulate comprising ticagrelor and pharmaceutically acceptable excipients;
b) providing a second granulate comprising acetylsalicylic acid and pharmaceutically acceptable excipients;
c) mixing the first granulate comprising ticagrelor and the second granulate comprising acetylsalicylic acid, thereby obtaining a third granulate comprising ticagrelor, acetylsalicylic acid, and pharmaceutically acceptable excipients; and
d) lubricating the third granulate.

Wherein stage a) of providing a first granulate comprising ticagrelor and pharmaceutically acceptable excipients comprises mixing and granulating the ticagrelor with a binder, a glidant, a wetting agent, and a diluent for at least 1 minute.

In a preferred embodiment, in the stage a) of providing a first granulate comprising ticagrelor and pharmaceutically acceptable excipients, high-shear wet granulation is carried out. The high-shear wet granulation process allows the high adhesion of ticagrelor to be reduced, thereby facilitating and making the subsequent stages and steps in the process for manufacturing the pharmaceutical composition more efficient.

As to stage b) of providing a second granulate comprising acetylsalicylic acid and pharmaceutically acceptable excipients, said stage comprises:
- mixing and granulating the acetylsalicylic acid with at least one of a binder, a wetting agent, and a diluent;
- heating the mixture and granulating at a temperature in the range of approximately 65 °C and 70 °C;
- adding and mixing a glidant, a wetting agent, and a diluent.

In a preferred embodiment, in the stage b) of providing a second granulate comprising acetylsalicylic acid and pharmaceutically acceptable excipients, high shear melt granulation is carried out. The high-shear melt granulation process prevents the presence of residual moisture or ambient moisture from influencing the degradation (hydrolysis) of acetylsalicylic acid.

In a non-limiting embodiment, the pharmaceutically acceptable excipients are as described below:
the diluent is selected from, but not limited to, the group consisting of starch derivatives such as pregelatinized starch and corn starch; cellulose derivatives such as microcrystalline cellulose; mannitol; maltitol; dextrose; dibasic calcium phosphate; and combinations thereof;
the binder is selected from, but not limited to, the group consisting of cellulose derivatives such as hydroxypropyl cellulose and carboxymethyl cellulose; starch derivatives such as pregelatinized starch and corn starch; polyvinylpyrrolidone; glyceryl dibehenate; povidones; polyethylene glycols; poloxamers; and combinations thereof;
the wetting agent is selected from, but not limited to, the group consisting of poloxamers; sodium lauryl sulfate; castor oil derivatives; benzalkonium chloride; benzetonium chloride; polyoxyethylene alkyl esters; polyoxyethylene sorbitan fatty acid esters; and combinations thereof;
the glidant is selected from, but not limited to, the group consisting of cellulose derivatives such as carboxymethyl cellulose and microcrystalline cellulose; starch derivatives such as pregelatinized starch and corn starch; silicate derivatives such as magnesium silicate and magnesium trisilicate; talc; colloidal silicon dioxide; and combinations thereof; and
the lubricant is selected from, but not limited to, the group consisting of stearate derivatives such as magnesium stearate, zinc stearate, calcium stearate, stearic acid, monostearates, and stearyl fumarate; sulfated derivatives such as magnesium lauryl sulfate and sodium lauryl sulfate; talc; and combinations thereof.

In a preferred embodiment, the process for manufacturing the pharmaceutical composition of the present invention comprises an additional encapsulating stage. Said encapsulating stage comprises filling capsules, preferably #0 capsules, with the wetted third granulate.

In an even more preferred embodiment, the process for manufacturing the pharmaceutical composition according to the present invention comprises:
I. Providing a first granulate comprising ticagrelor and pharmaceutically acceptable excipients:
   1. Mix and granulate ticagrelor (active ingredient) with a binder (hydroxypropyl cellulose), a glidant (colloidal silicon dioxide), a wetting agent (sodium lauryl sulfate), and a diluent (pregelatinized starch) for approximately 5 min. The excipients must be sifted previously.
   2. Add purified water, which evaporates during the process, and mix for approximately 5 min.
   3. Dry the granulate by microwave and vacuum for approximately 170 min until the moisture content of the granulate is between approximately 0.3% and 1.0%.
      In this stage, high-shear wet granulation is used.
II. Providing a second granulate comprising acetylsalicylic acid and pharmaceutically acceptable excipients:
   1. Mix and granulate acetylsalicylic acid (active ingredient), a first binder (glyceryl dibehenate), a wetting agent (sodium lauryl sulfate), a diluent (pregelatinized starch), and a second binder (hydroxypropyl cellulose) for approximately 3 minutes in a granulation equipment.
   2. Heat the granulation equipment containing the mixture from previous step 1 for approximately 15 minutes at a temperature of approximately 85 °C.
   3. Continue granulating the mixture until it reaches a temperature between approximately 65 °C and 70 °C.
   4. Add and mix a glidant (colloidal silicon dioxide), a wetting agent (sodium lauryl sulfate), and a diluent (microcrystalline cellulose PH112).
   In this stage, high-shear melt granulation is used.
III. Obtaining a third granulate comprising ticagrelor, acetylsalicylic acid, and pharmaceutically acceptable excipients:
   In this stage, the first granulate comprising ticagrelor (obtained in stage I) and the second granulate comprising acetylsalicylic acid (obtained in stage II) are mixed in a mixer for approximately 5 min.
IV. Incorporation of lubricant:
   The previously sifted lubricant (zinc stearate) is incorporated into the mixture obtained in stage III, i.e., the granulate comprising ticagrelor, acetylsalicylic acid, and pharmaceutically acceptable excipients. The process is carried out in a mixer for approximately 3 min.
   At the end of this stage, the pharmaceutical composition according to the present invention is obtained, which can be included in a capsule.
V. Encapsulation:
   For encapsulation, a #0 capsule is preferably used, which has an opaque orange body and an opaque orange cap.

Due to the novel process and formulation of the medicament, it was possible to obtain a product with demonstrably improved stability through studies conducted according to current regulatory guidelines, in which quality attributes were met during the assessment period. Stability testing is a means of comparing different formulations, packaging materials, or manufacturing processes in short-term experiments. Once the final formulation and manufacturing process have been established, the manufacturer conducts a series of stability tests to predict the product or medicament stability in this case and determine shelf-life and storage conditions thereof.

The present invention will be better understood from the following examples, which are presented solely for illustrative purposes to allow a thorough understanding of the preferred embodiments of the present invention, without implying that there are no other embodiments not illustrated that can be put into practice based on detailed description.

### EXAMPLES

### Example 1. Process for manufacturing a pharmaceutical composition according to one embodiment of the invention

Three batches of a pharmaceutical composition comprising ticagrelor at a dose of 90 mg and acetylsalicylic acid at a dose of 100 mg were manufactured, the qualitative and quantitative formula of which is detailed in Table 2.

**Table 2. Qualitative and quantitative formula of the pharmaceutical composition**

| **Components** | **Function in the formulation** | **Concentration in the formulation (mg/tablet)** |
|---|---|---|
| Ticagrelor | Drug | 90.00 |
| Acetylsalicylic acid | Drug | 100.00 |
| Pregelatinized starch | Diluent | 127.90 |
| Hydroxypropyl cellulose EF | Binder | 33.00 |
| Glyceryl dibehenate | Binder | 10.00 |
| Sodium lauryl sulfate | Wetting agent | 11.00 |
| Colloidal silicon dioxide | Glidant | 4.90 |
| Microcrystalline cellulose PH112 | Diluent | 74.20 |
| Zinc stearate | Lubricant | 3.00 |
| Purified water (mL) | Solvent | 0.075 |
| #0 Capsule with Opaque orange body and opaque orange cap | Vehicle | 96.00 |

To manufacture the pharmaceutical composition according to one embodiment of the present invention as described above, a process generally comprising four stages described below was carried out:
I. Providing a first granulate comprising ticagrelor and pharmaceutically acceptable excipients.
   In this stage, the following steps were carried out:
   1. Ticagrelor (drug) was mixed with 45% hydroxypropyl cellulose (binder), 50% colloidal silicon dioxide (glidant), 45% sodium lauryl sulfate (wetting agent), and 45% pregelatinized starch (diluent) in a granulation equipment for 5 min. The above components were previously sifted.
   2. In the granulation equipment, wetting was done with purified water while mixing for 5 minutes.
   3. In the granulation equipment, microwave vacuum drying was performed on the granulate for 170 min until the moisture content of the granulate was between 0.3-1.0%.
   4. A sieving operation was performed on the granulate using a 0.079-inch (2 mm) mesh.
II. Providing a second granulate comprising acetylsalicylic acid and pharmaceutically acceptable excipients.
   During this stage, the following steps were carried out:
   1. Acetylsalicylic acid (drug), glyceryl dibehenate (binder), 45% sodium lauryl sulfate (wetting agent), 40% pregelatinized starch (diluent), and 33% hydroxypropyl cellulose (binder) were mixed in a granulation equipment for 3 minutes. The above components were previously sifted.
   2. The granulator equipment was heated for 15 min at a temperature of 85 °C.
   3. Granulation of the mixture from step 1 was continued until a temperature between 65 °C and 70 °C was reached.
   4. 10% pregelatinized starch (diluent) and 22% hydroxypropyl cellulose (binder) were added to the granulation equipment.
   5. The granulation equipment was cooled to a temperature between 30 °C and 35 °C.
   6. The particle size of the granulate obtained in step 5 was reduced using a 0.050-inch (1.27 mm) mesh.
   7. 10% sodium lauryl sulfate (wetting agent) and microcrystalline cellulose PH112 (diluent) were added and mixed for 5 min.
III. Obtaining a third granulate comprising ticagrelor, acetylsalicylic acid, and pharmaceutically acceptable excipients.
   In this stage, the first granulate comprising ticagrelor (obtained in stage I) and the second granulate comprising acetylsalicylic acid (obtained in stage II) were mixed, the process was carried out in a mixer for approximately 5 min.
IV. Incorporation of lubricant
   The previously sifted lubricant (zinc stearate) was incorporated into the mixture obtained in stage III, i.e., the granulate comprising ticagrelor, acetylsalicylic acid, and pharmaceutically acceptable excipients. The process was carried out in a mixer for approximately 3 min.
V. Encapsulation
   A #0 capsule was used for encapsulation, which has an opaque orange body and an opaque orange cap.
   A capsule weighing 550.0 ± 45.0 mg was obtained with a disintegration time of less than 10 min in water at 37 °C ± 2.0 °C.

### Example 2. Stability of a pharmaceutical composition according to a preferred embodiment of the present invention

The batches of the pharmaceutical composition obtained in Example 1 were subjected to an accelerated stability study at a temperature of 40°C and a relative humidity of 75%.

The results of the study for the three batches are presented in Tables 3, 4, and 5.

**Table 3. Results of accelerated stability study for batch DFF2210-37**

| ANALYTICAL INFORM OF STABILITY | | | | | |
|---|---|---|---|---|---|
| TICAGRELOR - ACETYLSALICYLICIC ACID 90 mg/100 mg CAPSULES | | | | | |
| **CONDITION OF STABILITY STUDY:** 40°C/75%RH | | **PHARMACEUTICAL FORM:** CAPSULES | | | **BATCH:** DFF2210-37 |
| **ACTIVE INGREDIENT(S):** TICAGRELOR - ACETYLSALICYLIC ACID | | **DOSE:** 90mg/100mg | | | **PROJECT CODE:** NP21-002 |
| **STUDY START DATE:** December 30, 2022 | | **PACKAGE:** Cold-formed aluminum foil blister pack 176 µm - Aluminum foil 25 µm in white cardboard box | | | **VERSION:** 3.0 |
| | | | | | |

| **DETERMINATION** | **SPECIFICATION** | **INITIAL CONTROL** | **RESULT ACCORDING TO THE PERIOD OF STABILITY** | | |
|---|---|---|---|---|---|
| | | | **1 Month** | **3 Months** | **6 Months** |
| DESCRIPTION | #0 Capsule, opaque orange cap and body, containing beige powder, free of foreign particles. | #0 Capsule, opaque orange cap and body, containing beige powder, free of foreign particles. | #0 Capsule, opaque orange cap and body, containing beige powder, free of foreign particles. | #0 Capsule, opaque orange cap and body, containing beige powder, free of foreign particles. | #0 Capsule, opaque orange cap and body, containing beige powder, free of foreign particles. |
| TICAGRELOR CONTENT | Between 90.0% and 110.0% | 104.31% | 103.39% | 96.67% | 99.53% |
| | | 103.79% | 103.67% | 97.24% | 98.83% |
| | | 103.68% | 103.57% | 96.76% | 99.54% |
| | Between 81.0 mg and 99.0 mg / Capsule | **103.9%** | **103.5%** | **96.9%** | **99.3%** |
| | | 0.32% | 0.14% | 0.32% | 0.41% |
| | | 93.5 mg / Capsule | 93.2 mg / Capsule | 87.2 mg / Capsule | 89.4 mg / Capsule |
| ACETYLSALICYLIC ACID CONTENT | Between 90.0% and 110.0% | 95.28% | 97.36% | 93.00% | 95.13% |
| | | 96.01% | 98.43% | 94.15% | 95.03% |
| | | 94.26% | 98.05% | 91.83% | 95.88% |
| | Between 90.0 mg and 110.0 mg / Capsule | 95.2% | **97.9%** | **93.0%** | **95.4%** |
| | | 0.92% | 0.55% | 1.25% | 0.49% |
| | | 95.2 mg / Capsule | 97.9 mg / Capsule | 93.0 mg / Capsule | 95.4 mg / Capsule |
| TICAGRELOR DISSOLUTION | Q = 60.00% in 60 minutes | 92.90% | 82.42% | 80.66% | 82.05% |
| | | 99.93% | 89.56% | 102.46% | 86.05% |
| | | 104.74% | 91.90% | 91.15% | 84.04% |
| | | 105.54% | 84.04% | 94.50% | 91.29% |
| | | 97.56% | 90.31% | 96.13% | 93.51% |
| | | 95.42% | 91.09% | 99.36% | 82.81% |
| | | **99.35%** | **88.22%** | **94.04%** | **86.62%** |
| | | 5.09% | 4.51% | 8.12% | 5.46% |
| ACETYLSALICYLIC ACID DISSOLUTION | Q = 60.00% in 45 minutes | 84.28% | 86.67% | 80.35% | 75.90% |
| | | 84.44% | 91.01% | 101.89% | 86.70% |
| | | 87.90% | 93.05% | 82.33% | 77.77% |
| | | 87.85% | 92.84% | 88.45% | 82.04% |
| | | 86.50% | 91.66% | 81.35% | 68.36% |
| | | 79.18% | 93.60% | 84.96% | 78.36% |
| | | **85.02%** | **91.47%** | **86.56%** | **78.19%** |
| | | 3.85% | 2.77% | 9.31% | 7.87% |
| ORGANIC IMPURITIES TICAGRELOR | KNOWN IMPURITY 1 | No detected | 0.22% | 0.40% | 0.53% |
| | No more than 4.00% | | | | |
| ORGANIC IMPURITIES TICAGRELOR | KNOWN IMPURITY 2 | No detected | 0.43% | 0.76% | 1.28% |
| | No more than 4.00% | | | | |
| ORGANIC IMPURITIES TICAGRELOR | INDIVIDUAL IMPURITIES | 0.002% | 0.037% | 0.033% | 0.029% |
| | No more than 0.200% | | | | |
| ORGANIC IMPURITIES TICAGRELOR | TOTAL IMPURITIES | 0.007% | 0.131% | 0.132% | 0.150% |
| | No more than 1.200% | | | | |
| SALICYLIC ACID LIMIT | No more than 90.0% | 0.307% | 2.403% | 5.600% | 5.210% |
| | | 0.277% | 2.297% | 5.420% | 5.330% |
| | | 0.310% | 2.321% | 5.770% | 5.050% |
| | | **0.30%** | **2.34%** | **5.60%** | **5.19%** |
| | | 6.06% | 2.39% | 3.16% | 2.65% |

**Table 4. Results of accelerated stability study for batch DFF2210-38**

| ANALYTICAL INFORM OF STABILITY | | | | | |
|---|---|---|---|---|---|
| TICAGRELOR - ACETYLSALICYLIC ACID 90 mg/100 mg CAPSULES | | | | | |
| **CONDITION OF STABILITY STUDY:** 40°C/75%RH | | **PHARMACEUTICAL FORM:** CAPSULES | | | **BATCH:** DFF2210-38 |
| **ACTIVE INGREDIENT(S):** TICAGRELOR - ACETYLSALICYLIC ACID | | **DOSE:** 90mg/100mg | | | **PROJECT CODE:** NP21-002 |
| **STUDY START DATE:** December 30, 2022 | | **PACKAGE:** Cold-formed aluminum foil blister pack 176 µm - Aluminum foil 25 µm in white cardboard box | | | **VERSION:** 3.0 |
| | | | | | |

| **DETERMINATION** | **SPECIFICATION** | **INITIAL CONTROL** | **RESULT ACCORDING TO THE PERIOD OF STABILITY** | | |
|---|---|---|---|---|---|
| | | | **1 Month** | **3 Months** | **6 Months** |
| DESCRIPTION | #0 Capsule, opaque orange cap and body, containing beige powder, free of foreign particles. | #0 Capsule, opaque orange cap and body, containing beige powder, free of foreign particles. | #0 Capsule, opaque orange cap and body, containing beige powder, free of foreign particles. | #0 Capsule, opaque orange cap and body, containing beige powder, free of foreign particles. | #0 Capsule, opaque orange cap and body, containing beige powder, free of foreign particles. |
| TICAGRELOR CONTENT | Between 90.0% and 110.0% | 105.72% | 101.49% | 100.81% | 99.01% |
| | | 103.21% | 101.19% | 101.01% | 100.27% |
| | | 106.65% | 102.23% | 102.57% | 99.28% |
| | Between 81.0 mg and 99.0 mg / Capsule | 105.2% | 101.6% | 101.5% | 99.5% |
| | | 1.69% | 0.53% | 0.95% | 0.66% |
| | | 94.7 ma / Capsule | 91.5 ma / Capsule | 91.3 mg / Capsule | 89.6 ma / Capsule |
| ACETYLSALICYLIC ACID CONTENT | Between 90.0% and 110.0% | 96.35% | 99.51% | 101.30% | 97.25% |
| | | 95.87% | 99.17% | 98.91% | 97.71% |
| | | 96.18% | 99.37% | 102.47% | 98.48% |
| | Between 90.0 mg and 110.0 mg / Capsule | 96.1% | 99.3% | 100.9% | 97.8% |
| | | 0.25% | 0.17% | 1.80% | 0.64% |
| | | 96.1 ma / Capsule | 99.4 ma / Capsule | 100.9 ma / Capsule | 97.8 ma / Capsule |
| TICAGRELOR DISSOLUTION | Q = 60.00% in 60 minutes | 93.83% | 89.29% | 89.85% | 83.51% |
| | | 93.23% | 90.02% | 83.73% | 85.88% |
| | | 96.97% | 98.57% | 87.71% | 83.02% |
| | | 107.92% | 96.98% | 93.38% | 82.93% |
| | | 109.26% | 95.12% | 86.03% | 85.29% |
| | | 100.56% | 91.64% | 91.90% | 94.13% |
| | | 100.30% | 93.60% | 88.77% | 85.79% |
| | | 6.93% | 4.10% | 4.10% | 4.97% |
| ACETYLSALICYLIC ACID DISSOLUTION | Q = 60.00% in 45 minutes | 90.27% | 82.44% | 85.58% | 80.27% |
| | | 81.26% | 81.15% | 89.49% | 69.18% |
| | | 86.24% | 75.63% | 86.46% | 66.85% |
| | | 88.33% | 80.87% | 85.24% | 73.20% |
| | | 86.47% | 76.25% | 75.38% | 75.39% |
| | | 81.40% | 77.69% | 83.65% | 80.18% |
| | | 85.66% | 79.00% | 84.30% | 74.18% |
| | | 4.27% | 3.61% | 5.67% | 7.49% |
| ORGANIC IMPURITIES TICAGRELOR | KNOWN IMPURITY 1 | No detected | 0.26% | 0.47% | 0.53% |
| | No more than 4.00% | | | | |
| ORGANIC IMPURITIES TICAGRELOR | KNOWN IMPURITY 2 | No detected | 0.42% | 0.79% | 1.41% |
| | No more than 4.00% | | | | |
| ORGANIC IMPURITIES TICAGRELOR | INDIVIDUAL IMPURITIES | 0.002% | 0.034% | 0.032% | 0.034% |
| | No more than 0.200% | | | | |
| ORGANIC IMPURITIES TICAGRELOR | TOTAL IMPURITIES | 0.006% | 0.115% | 0.139% | 0.167% |
| | No more than 1.200% | | | | |
| SALICYLIC ACID LIMIT | No more than 90.0% | 0.317% | 2.608% | 6.030% | 7.600% |
| | | 0.285% | 2.529% | 5.890% | 7.710% |
| | | 0.318% | 2.544% | 5.870% | 7.610% |
| | | 0.31% | 2.56% | 5.93% | 7.64% |
| | | 6.07% | 1.64% | 1.46% | 0.76% |

**Table 5. Results of accelerated stability study for batch DFF2210-39**

| ANALYTICAL INFORM OF STABILITY | | | | | |
|---|---|---|---|---|---|
| TICAGRELOR - ACETYLSALICYLIC ACID 90 mg/100 mg CAPSULES | | | | | |
| **CONDITION OF STABILITY STUDY:** 40°C/75%RH | | **PHARMACEUTICAL FORM:** CAPSULES | | | **BATCH:** DFF2210-39 |
| **ACTIVE INGREDIENT(S):** TICAGRELOR-ACETYLSALICYLIC ACID | | DOSE: 90mg/100mg | | | **PROJECT CODE:** NP21-002 |
| **STUDY START DATE:** December 30, 2022 | | **PACKAGE:** Cold-formed aluminum foil blister pack 176 µm - Aluminum foil 25 µm in white cardboard box | | | **VERSION:** 3.0 |
| | | | | | |

| **DETERMINATION** | **SPECIFICATION** | **INITIAL CONTROL** | **RESULT ACCORDING TO THE PERIOD OF STABILITY** | | |
|---|---|---|---|---|---|
| | | | **1 Month** | **3 Months** | **6 Months** |
| DESCRIPTION | #0 Capsule, opaque orange cap and body, containing beige powder, free of foreign particles. | #0 Capsule, opaque orange cap and body, containing beige powder, free of foreign particles. | #0 Capsule, opaque orange cap and body, containing beige powder, free of foreign particles. | #0 Capsule, opaque orange cap and body, containing beige powder, free of foreign particles. | #0 Capsule, opaque orange cap and body, containing beige powder, free of foreign particles. |
| TICAGRELOR CONTENT | 98.80% Between 90.0% and 110.0% | 98.62% | 99.57% 99.98% | 101.31% 99.73% | 98.77% 99.16% |
| | | 98.81% | 99.78% | 99.33% | 99.12% |
| | Between 81.0 mg and 99.0 mg / Capsule | 98.7% | 99.8% | 100.1% | 99.0% |
| | | 0.11% | 0.20% | 1.05% | 0.22% |
| | | 88.8 ma / Capsule | 89.8 ma / Capsule | 90.11 ma / Capsule | 89.1 ma / Capsule |
| ACETYLSALICYLIC ACID CONTENT | Between 90.0% and 110.0% | 97.69% | 101.10% | 99.89% | 97.63% |
| | | 97.50% | 100.01% | 97.56% | 96.71% |
| | | 97.93% | 99.62% | 97.43% | 97.65% |
| | Between 90.0 mg and 110.0 mg / Capsule | 97.7% | 100.2% | 98.3% | 97.3% |
| | | 0.22% | 0.77% | 1.41% | 0.55% |
| | | 97.7 ma / Capsule | 100.2 ma / Capsule | 98.3 ma / Capsule | 97.3 ma / Capsule |
| TICAGRELOR DISSOLUTION | Q = 60.00% in 60 minutes | 89.16% | 93.36% | 97.90% | 87.58% |
| | | 88.34% | 95.86% | 87.42% | 85.73% |
| | | 92.00% | 90.72% | 83.62% | 82.42% |
| | | 93.93% | 100.28% | 95.08% | 81.38% |
| | | 123.61% | 95.40% | 94.06% | 82.48% |
| | | 95.54% | 101.52% | 95.46% | 82.99% |
| | | 97.10% | 96.19% | 92.26% | 83.76% |
| | | 13.67% | 4.25% | 5.96% | 2.83% |
| ACETYLSALICYLIC ACID DISSOLUTION | Q = 60.00% in 45 minutes | 93.81% | 82.03% | 88.82% | 69.95% |
| | | 93.08% | 91.02% | 78.66% | 70.71% |
| | | 84.42% | 90.03% | 84.91% | 80.11% |
| | | 93.94% | 92.71% | 85.67% | 68.85% |
| | | 91.67% | 95.87% | 84.77% | 87.58% |
| | | 91.66% | 91.46% | 74.01% | 78.62% |
| | | 91.43% | 90.52% | 82.81% | 75.97% |
| | | 3.91% | 5.11% | 6.55% | 9.74% |
| ORGANIC IMPURITIES TICAGRELOR | KNOWN IMPURITY 1 | No detected | 0.28% | 0.46% | 0.54% |
| | No more than 4.00% | | | | |
| ORGANIC IMPURITIES TICAGRELOR | KNOWN IMPURITY 2 | No detected | 0.44% | 0.81% | 1.43% |
| | No more than 4.00% | | | | |
| ORGANIC IMPURITIES TICAGRELOR | INDIVIDUAL IMPURITIES | 0.004% | 0.036% | 0.033% | 0.034% |
| | No more than 0.200% | | | | |
| ORGANIC IMPURITIES TICAGRELOR | TOTAL IMPURITIES | 0.010% | 0.124% | 0.142% | 0.169% |
| | No more than 1.200% | | | | |
| SALICYLIC ACID LIMIT | No more than 9.00% | 0.284% | 2.624% | 6.040% | 7.540% |
| | | 0.291% | 2.576% | 6.270% | 7.520% |
| | | 0.301% | 2.665% | 6.270% | 7.640% |
| | | **0.29%** | **2.62%** | **6.19%** | **7.57%** |
| | | 3.02% | 1.70% | 2.21% | 0.82% |

From the above Tables it is clear that the pharmaceutical composition according to the present invention is stable, as the batches tested met the specifications during the 6 months of analysis.

### Example 3. Dissolution profiles

During the development of the pharmaceutical composition of the present invention, the dissolution profile characterization of each drug in the proposed combination was performed in comparison with the monodrug (reference medicament). The results obtained are shown below:
a) Acetylsalicylic acid
   Dissolution conditions
   Apparatus: 1 (baskets)
   Dissolution medium: 0.05 M acetate buffer solution, pH 4.5 ± 0.05, degassed
   Medium temperature: 37.0 °C ± 0.5 °C
   Medium volume: 500 mL
   Stirring speed: 100 rpm
   Sampling times: 45 min every 5 min; for characterization purposes, the evaluation was also performed at 60 min.

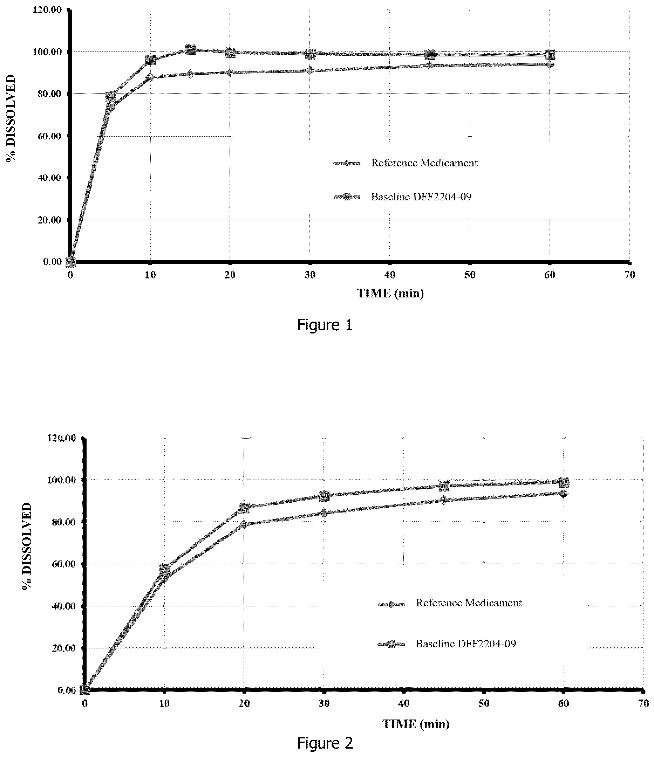

   Figure 1 depicts the dissolution profile of acetylsalicylic acid. From said profile, it is clear that the trend of the pharmaceutical composition according to the present invention is similar to that of the reference medicament. It is noteworthy that at 10 min, a greater amount of drug is dissolved in the pharmaceutical composition according to the present invention. It follows from the above that the present invention allows the solubility of acetylsalicylic acid to be improved.
b) Ticagrelor
   Dissolution conditions
   Apparatus: 2 (Paddles)
   Dissolution medium: 0.2% (m/v) polysorbate 80 solution in water
   Medium temperature: 37.0 °C ± 0.5 °C
   Medium volume: 900 mL
   Stirring speed: 75 rpm
   Sampling times: 60 min every 10 min

Figure 2 shows the dissolution profile of ticagrelor. From said profile, it is clear that the trend of the pharmaceutical composition according to the present invention is similar to that of the reference medicament. It is noteworthy that at 20 min, a greater amount of the drug is dissolved in the pharmaceutical composition according to the present invention. It follows from the above that the present invention allows the solubility of ticagrelor to be improved.

According to the matter described above, it can be seen that the pharmaceutical composition comprising ticagrelor and acetylsalicylic acid has been conceived to provide a stable, immediate-release pharmaceutical form, and it will be evident to any person skilled in the art that embodiments with regard to a pharmaceutical composition comprising ticagrelor and acetylsalicylic acid, process for preparing the same, and uses thereof as described above and illustrated in the accompanying drawings are only illustrative and not limiting of the present invention, since numerous changes of consideration are possible in their details without departing from the scope of the invention.

Therefore, the present invention shall not be considered as restricted except for what is required by prior art and the scope of the attached claims.

## Claims

1. A pharmaceutical composition **characterized in that** it comprises:
- ticagrelor at a dose of 90 mg;
- acetylsalicylic acid at a dose of from 75 to 100 mg;
- at least one diluent in an amount of from 5 to 30% by weight of the composition;
- at least one binder in an amount of from 1 to 8% by weight of the composition;
- a wetting agent in an amount of from 1 to 2.5% by weight of the composition;
- a glidant in an amount of from 0.5% to 2% by weight of the composition; and
- a lubricant in an amount of from 0.5% to 1.5% by weight of the composition.

2. The pharmaceutical composition according to claim 1, wherein the at least one diluent is selected from the group consisting of starch derivatives such as pregelatinized starch and corn starch; cellulose derivatives such as microcrystalline cellulose; mannitol; maltitol; dextrose; dibasic calcium phosphate; and combinations thereof.

3. The pharmaceutical composition according to claim 2, wherein the composition comprises two diluents.

4. The pharmaceutical composition according to claim 1, wherein the at least one binder is selected from the group consisting of cellulose derivatives such as hydroxypropyl cellulose and carboxymethyl cellulose; starch derivatives such as pregelatinized starch and corn starch; polyvinylpyrrolidone; glyceryl dibehenate; povidones; polyethylene glycols; poloxamers; and combinations thereof.

5. The pharmaceutical composition according to claim 4, wherein the composition comprises two binders.

6. The pharmaceutical composition according to claim 1, wherein the wetting agent is selected from the group consisting of poloxamers; sodium lauryl sulfate; castor oil derivatives; benzalkonium chloride; benzetonium chloride; polyoxyethylene alkyl esters; polyoxyethylene sorbitan fatty acid esters; and combinations thereof.

7. The pharmaceutical composition according to claim 1, wherein the glidant is selected from the group consisting of cellulose derivatives such as carboxymethyl cellulose and microcrystalline cellulose; starch derivatives such as pregelatinized starch and corn starch; silicate derivatives such as magnesium silicate and magnesium trisilicate; talc; colloidal silicon dioxide; and combinations thereof.

8. The pharmaceutical composition according to claim 1, wherein the lubricant is selected from the group consisting of stearate derivatives such as magnesium stearate, zinc stearate, calcium stearate, stearic acid, monostearates, and stearyl fumarate; sulfated derivatives such as magnesium lauryl sulfate and sodium lauryl sulfate; talc; and combinations thereof.

9. The pharmaceutical composition according to any of claims 1 to 8, wherein it comprises:
- ticagrelor at a dose of 90 mg;
- acetylsalicylic acid at a dose of from 75 to 100 mg;
- pregelatinized starch as a first diluent in an amount of from 20% to 30% by weight of the composition;
- hydroxypropyl cellulose as a first binder in an amount of from 2% to 3% by weight of the composition;
- glyceryl dibehenate as a second binder in an amount of from 1% to 2% by weight of the composition;
- sodium lauryl sulfate as a wetting agent in an amount of from 1.5% to 2.5% by weight of the composition;
- colloidal silicon dioxide as a glidant in an amount of from 0.5% to 2% by weight of the composition;
- microcrystalline cellulose as a second diluent in an amount of from 10% to 15% by weight of the composition; and
- zinc stearate as a lubricant in an amount of from 0.5% to 1% by weight of the composition.

10. The pharmaceutical composition according to any of claims 1 to 9, wherein the pharmaceutical composition is in the pharmaceutical form of capsule.

11. The pharmaceutical composition according to claim 10, wherein it comprises a pharmaceutically acceptable vehicle.

12. The pharmaceutical composition according to claim 11, wherein the pharmaceutically acceptable vehicle is a capsule.

13. A process for manufacturing a pharmaceutical composition as described in claims 1 to 12, **characterized in that** it comprises the following stages:
a) providing a first granulate comprising ticagrelor and pharmaceutically acceptable excipients;
b) providing a second granulate comprising acetylsalicylic acid and pharmaceutically acceptable excipients;
c) mixing the first granulate comprising ticagrelor and the second granulate comprising acetylsalicylic acid, thereby obtaining a third granulate comprising ticagrelor, acetylsalicylic acid, and pharmaceutically acceptable excipients; and
d) lubricating the third granulate.

14. The process according to claim 13, wherein the stage a) of providing a first granulate comprising ticagrelor and pharmaceutically acceptable excipients comprises mixing and granulating the ticagrelor with a binder, a glidant, a wetting agent, and a diluent for at least 5 min.

15. The process according to claim 14, wherein in the stage a) of providing a first granulate comprising ticagrelor and pharmaceutically acceptable excipients, high-shear wet granulation is carried out.

16. The process according to claim 13, wherein the stage b) of providing a second granulate comprising acetylsalicylic acid and pharmaceutically acceptable excipients comprises:
- mixing and granulating the acetylsalicylic acid with at least one of a binder, a wetting agent, and a diluent;
- heating the mixture and granulating at a temperature in the range of 65 °C and 70 °C;
- adding and mixing a glidant, a wetting agent, and a diluent.

17. The process according to claim 16, wherein in the stage b) of providing a second granulate comprising acetylsalicylic acid and pharmaceutically acceptable excipients, high-shear melt granulation is carried out.

18. The process according to any of claims 13 to 17, wherein:
the diluent is selected from the group consisting of starch derivatives such as pregelatinized starch and corn starch; cellulose derivatives such as microcrystalline cellulose; mannitol; maltitol; dextrose; dibasic calcium phosphate; and combinations thereof;
the binder is selected from the group consisting of cellulose derivatives such as hydroxypropyl cellulose and carboxymethyl cellulose; starch derivatives such as pregelatinized starch and corn starch; polyvinylpyrrolidone; glyceryl dibehenate; povidones; polyethylene glycols; poloxamers; and combinations thereof;
the wetting agent is selected from the group consisting of poloxamers; sodium lauryl sulfate; castor oil derivatives; benzalkonium chloride; benzetonium chloride; polyoxyethylene alkyl esters; polyoxyethylene sorbitan fatty acid esters; and combinations thereof;
the glidant is selected from the group consisting of cellulose derivatives such as carboxymethyl cellulose and microcrystalline cellulose; starch derivatives such as pregelatinized starch and corn starch; silicate derivatives such as magnesium silicate and magnesium trisilicate; talc; colloidal silicon dioxide; and combinations thereof; and
the lubricant is selected from the group consisting of stearate derivatives such as magnesium stearate, zinc stearate, calcium stearate, stearic acid, monostearates, and stearyl fumarate; sulfated derivatives such as magnesium lauryl sulfate and sodium lauryl sulfate; talc; and combinations thereof.

19. The process according to any of claims 13 to 18, wherein it comprises an additional stage of encapsulation.

20. A composition as claimed in any of claims 1 to 12 for use in the prevention, treatment, or control of a cardiovascular disease.

21. The composition for use according to claim 20, wherein the cardiovascular disease is selected from the group consisting of coronary heart disease, heart failure, arrhythmia, cerebrovascular disease, peripheral artery disease, congenital heart disease, cardiomyopathy, valvular heart disease, and for angioplasty procedures.
